# FASCICULE DE BREVET EUROPEEN

(11) **EP 0 717 997 B1**
(45) Date de publication et mention de la délivrance du brevet: **16.08.2001**
(21) Numéro de dépôt: 95402551.6
(22) Date de dépôt: 15.11.1995
(51) Int. Cl.: A61K 38/08, A61K 38/10, A61K 31/395, A61K 7/48

(54) **Utilisation d'un antagoniste de substance P dans une composition pharmaceutique**
Verwendung von Substanz P Antagonist im pharmazeutischer Zusammensetzung
Use of substance P antagonist in pharmaceutical composition

(30) Priorité: 19.12.1994 FR 9415251
(43) Date de publication de la demande: 26.06.1996
(73) Titulaire: L'OREAL, 75008 Paris (FR)
(72) Inventeur: De Lacharriere, Olivier, F-75015 Paris (FR); Breton, Lionel, F-78000 Versailles (FR)
(74) Mandataire: Lhoste, Catherine

(56) Documents cités:
- EP-A- 0 515 681
- WO-A-93/06099
- DATABASE WPI Section Ch, Week 9404 Derwent Publications Ltd., London, GB; Class B02, AN 94-031791 & JP-A-05 339 240 ( YAMANOUCHI PHARM CO LTD) , 21 Décembre 1993
- DATABASE WPI Section Ch, Week 9433 Derwent Publications Ltd., London, GB; Class B05, AN 94-269449 & JP-A-06 199 892 ( TAKEDA CHEM IND LTD) , 19 Juillet 1994
- PROCEEDINGS OF THE NATIONAL ACADEMY OF SCIENCES OF USA, vol. 87, no. 12, 1 Juin 1990 pages 4833-4835, XP 000134336 FOLKERS K ET AL 'SPANTIDE II, AN EFFECTIVE TACHYKININ ANTAGONIST HAVING HIGH POTENCY AND NEGLIGIBLE NEUROTOXICITY'

## Description

La présente invention se rapporte à l'utilisation d'un antagoniste de substance P pour la préparation d'une composition pharmaceutique, pour traiter certaines maladies de la peau d'origine neurogène.

Plus spécialement, cette composition permet le traitement par voie topique, ingérable ou injectable des lichens notamment les lichens plans et les lichens pigmentaires ; des prurigos et en particulier les prurigos actinique, de Besnier ou Hebra ou strophulus ou de Hyde ; des toxidermies prurigineuses et les prurits sévères d'origine neurogène, en particulier ceux des hémodialysés, des sidéens, les prurits cholestatiques ou biliaires. Les toxidermies prurigineuses sont notamment consécutives à l'absorption de médicament ; ces maladies sont très différentes de l'urticaire et ne font intervenir aucune réaction de contact.

Jusqu'à ce jour, les lichens plans et pigmentaires étaient traités à l'aide de corticoïdes locaux ou de puvathérapie. Les corticoïdes sont certes très efficaces pour calmer les symptômes mais malheureusement, ils présentent des effets secondaires souvent très pénalisants comme des atrophies, des infections surtout mycosiques ou bactériennes. La puvathérapie est l'irradiation locale de la peau malade avec des UVA, après absorption d'une substance photosensibilisante (psoralène). Cette technique présente les inconvénients graves d'un photovieillissement pouvant entraîner le plus souvent des cancers de la peau. De plus, ce traitement n'est pas ambulatoire, obligeant les malades à se rendre courramment dans un centre spécialisé pendant toute la durée du traitement, ce qui est très contraignant et limite leur activité professionnelle.

Les prurigos sont aussi traités par les corticoïdes locaux, puvathérapie ou thalidomide. Les corticoïdes locaux et la puvathérapie présentent les inconvénients ci-dessus. La thalidomide présente l'inconvénient majeur d'être tératogène, ce qui interdit son utilisation chez la femme enceinte. De plus, sa préscription très réglementée (limitée aux médecins hospitaliers) limite son emploi.

Les toxidermies prurigineuses sont actuellement traitées au moyen de corticoïdes locaux et/ou d'anti-histaminiques : leur traitement présente donc les mêmes inconvénients que ceux indiqués ci-dessus.

Les prurits sévères sont aussi traitées avec des corticoïdes locaux avec les mêmes inconvénients que ceux indiqués ci-dessus.

En outre, il est connu du document Merck manual, 16^{ème} éd., 1992 (p. 2406-7), que les prurits peuvent accompagner des maladies cutanées et se manifester en cas de peaux sèches ou de lésions de la peau. Ce document enseigne en outre, d'hydrater la peau par application d'émollients et de traiter les prurits par administration d' antihistaminiques.

La présente invention a justement pour objet l'utilisation, dans un milieu pharmaceutiquement ou dermatologiquement acceptable, d'un ou plusieurs antagonistes de substances P permettant de traiter efficacement certaines maladies de peau, tout en remédiant aux inconvénients mentionnés ci-dessus.

La substance P est un élément chimique polypeptidique élaboré et libéré par une terminaison nerveuse. Elle fait partie de la famille des tachykinines. La substance P intervient notamment dans la transmission de la douleur et dans des maladies du système nerveux central tels que l'anxiété, la schizophrénie, dans des maladies respiratoires et inflammatoires, dans des maladies gastrointestinales, dans des maladies rhumatismales et dans certaines maladies cutanées comme l'eczéma, le psoriasis, l'urticaire et les dermites de contact.

Il est connu d'utiliser les antagonistes de substance P pour traiter ces maladies. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808, WO-A-93/01165, WO-A-93/10073 et WO-A-94/08997.

Cependant personne n'avaient jusqu'à ce jour envisagé de traiter les lichens, les prurigos, les toxidermies prurigineuses et les prurits sévères d'origine neurogène au moyen d'antagonistes de substance P.

Certains des documents cités ci-dessus, ainsi que les documents JP-05-339240 et JP-06-199892 décrivent une grande quantité de maladies pouvant être traitées par des compositions contenant des antagonistes de substance P, et entre autres, certaines des maladies cutanées appelées aussi dermatoses. L'invention se rapporte au traitement symptomatique (démangeaisons, irritations) d'une dermatose et non au traitement de la maladie en elle-même. Ainsi, l'invention se rapporte à l'utilisation d'un antagoniste de substance P pour traiter l'inconfort associé à certaines dermatoses, et en particulier à l'eczéma et à la dermatite atopique, et non au traitement des autres signes de ces dermatoses (vésicules, érythème, etc).

Par ailleurs, les maladies cutanées citées dans ces documents sont des allergies et non des maladies d'origine neurogène. Or, l'invention ne concerne pas le traitement des allergies. En effet, l'allergie est un processus immunologique qui ne se produit que lorsqu'un allergène est présent et qui ne touche que certains sujets sensibilisés, alors que les maladies d'origine neurogène peuvent concerner tout individu.

Par conséquent, les documents cités ci-dessus ne décrivent ni ne suggèrent nullement l'emploi d'antagonistes de substance P pour traiter les symptômes d'origine neurogène.

La présente invention a donc pour objet l'utilisation d'au moins un antagoniste de substance P pour la préparation d'une composition pharmaceutique ou dermatologique pour le traitement symptomatique des lichens, des prurigos, des toxidermies prurigineuses et des prurits sévères d'origine neurogène.

La composition de l'invention contient un milieu pharmaceutiquement ou dermatologiquement acceptable, c'est-à-dire un milieu compatible avec les tissus, les muqueuses, la peau, les ongles et les cheveux. Ainsi, la composition contenant un ou plusieurs antagonistes de substance P peut être injectée, ingérée ou appliquée sur la peau notamment du visage, du cou, des cheveux, des ongles, des grands plis ou de toute autre zone cutanée du corps, les muqueuses (buccale, jugale, gingivale, génitale, anale).

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action.

L'antagoniste de substance P de l'invention peut être notamment un peptide ou un dérivé non peptidique, et plus précisément un composé comportant un hétérocycle azoté, soufré ou oxygéné, ou un composé comprenant un atome d'azote lié directement ou indirectement à un cycle benzènique.

On peut utiliser dans l'invention par exemple comme peptide antagoniste de substance P le sendide et le spantide II.

Le sendide correspond à la formule : Tyr D-Phe Phe D-His Leu Met NH2 dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule : D-NicLys Pro 3-Pal Pro D-Cl2Phe Asn D-Trp Phe D-Trp Leu Nle NH2 dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un hétéroatome lié directement ou indirectement à un cycle benzénique ou contenu dans un hétérocycle. En particulier cet hétéroatome est un atome d'oxygène, d'azote ou de soufre.

Comme composé hétérocyclique comportant un atome d'azote, on peut utiliser dans l'invention ceux décrits dans les documents suivants,: EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, WO-A-94/08997.

En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Les composés comportant un atome de soufre ou d'oxygène, utilisables dans l'invention sont notamment les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et EP-A-299457, et plus spécialement les alcoxy- et/ou aryloxy-tétrazolyl-benzofuranne-carboxamides ou les alcoxy- et/ou aryloxy-tétrazolyl-benzothiophène-carboxamides.

Comme composés comportant un atome d'azote lié directement ou indirectement à un noyau benzénique, on peut citer ceux décrits dans les documents suivants : EP-A-522808, WO-A-93/10073 et WO-A-93/01165. En particulier on peut citer les dérivés de l'éthylène diamine comme la N,N'bis-di-(3,5-diméthylbenzyl) éthylène diamine ou la N,N'bis-di-(3,5-diméthoxylbenzyl) éthylène diamine. Ces composés sont décrits comme intermédiaire de réactions dans le document WO-A-93/11338 déposé au nom de la demanderesse.

Les antagonistes de substance P peuvent être synthétisés ou extraits de produits naturels (végétaux ou animaux).

Dans les compositions de l'invention, l'antagoniste de substance P peut être utilisé de préférence en une quantité allant de 0,000001 à 5 % en poids par rapport au poids total de la composition, et en particulier en une quantité allant de 0,0001 à 0,1 % en poids par rapport au poids total de la composition.

Les compositions de l'invention peuvent se présenter sous toutes les formes galéniques normalement utilisées selon que la composition doit être ingérée, injectée ou appliquée sur la peau ou les muqueuses.

Pour une application topique, la composition peut se présenter sous forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, des microémulsions ou encore de microcapsules, microparticules, ou de dispersions vésiculaires de type ionique et/ou non ionique. Ces compositions sont préparées selon les méthodes usuelles.

Elle peut également être utilisée pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Pour l'injection, la composition peut se présenter sous forme d'une lotion aqueuse, huileuse ou sous forme d'un sérum.

Pour l'ingestion, la composition peut se présenter sous forme de capsules,de sirops, de granulés ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, des laits corporels de protection ou de soin, des lotions, gels ou mousses pour le soin de la peau ou des muqueuses, comme des lotions de nettoyage ou de désinfection, des compositions pour le bain, des compositions contenant un agent bactéricide.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Lorsque la composition de l'invention est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou gel huileux, la quantité d'huile peut aller jusqu'à plus de 90 % du poids total de la composition.

De façon connue, la composition pharmaceutique ou dermatologique de l'invention peut contenir également des adjuvants habituels dans les domaines considérés, tels que les gélifiants hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers). On peut ajouter à ces huiles des alcools gras et des acides gras (acide stéarique). On peut aussi utiliser des cires comme la cire d'abeille, de carnauba ou la paraffine.

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Il est en outre possible d'introduire des actifs hydrophiles comme les protéines ou les hydrolysats de protéine, les acides aminés, les polyols, l'urée, les sucres et les dérivés de sucre, les vitamines, l'amidon, les extraits végétaux(Aloe Vera) et les hydroxyacides (citrique, lactique, glycolique, tartrique).

On peut encore introduire des actifs lipophiles comme le rétinol (vitamine A) et ses dérivés les rétinoïdes comme l'acide rétinoïque 13 cis ou tout trans, le tocophérol (vitamine E) et ses dérivés, les acides gras essentiels, les céramides, les huiles essentielles, l'acide salicylique et ses dérivés (n-octanoyl-5-salicylique). Les acide salicyliques, lactique, acétique etc. servent notamment d'antiseptiques.

Les compositions suivantes illustrent l'invention : les proportions indiquées sont des pourcentages en poids.

### Composition 1 : Lotion désinfectante pour le visage ou les muqueuses

| | |
|---|---|
| N,N'-bis-di-(3,5-diméthoxybenzyle) éthylène diamine | 5,00 |
| Antioxydant | 0,05 |
| Isopropanol | 40,00 |
| Conservateur | 0,30 |
| Eau | qsp 100 % |

Cette composition peut être utiliséer aussi bien pour traiter les lichens plans ou pigmentaires ainsi que les toxidermies prurigineuses.

### Composition 2 : Gel pour le visage ou le corps pour le traitement du lichen plan

| | |
|---|---|
| N,N'-bis-di-(3,5-diméthoxybenzyle) éthylène diamine Hydroxypropylcellulose (Klucel H® vendu par la société | 0,05 |
| Hercules) | 1,00 |
| Acide salicylique | 1,00 |
| Antioxydant | 0,05 |
| Isopropanol | 40,00 |
| Conservateur | 0,30 |
| Eau | qsp 100 % |

### Composition 3 : Crème (émulsion huile-dans-eau) pour le traitement des prurits sévères

| | |
|---|---|
| N,N'-bis-di-(3,5-diméthoxybenzyle) éthylène diamine | 0,2 |
| Stéarate de glycérol | 2,00 |
| Acide lactique/acide acétique | 1,00 |
| Polysorbate 60 (Tween 60® vendu par la société ICI) | 1,00 |
| Acide stéarique | 1,40 |
| Triéthanolamine | 0,70 |
| Carbomère | 0,40 |
| Huile siliconée | 12,00 |
| Perhydrosqualène | 12,00 |
| Antioxydant | 0,05 |
| Parfum | 0,5 |
| Conservateur | 0,30 |
| Eau | qsp 100 % |

### Composition 4 : Gel pour le traitement des lichens

| | |
|---|---|
| Sendide | 5,00 |
| Hydroxypropylcellulose (Klucel H®) | 1,00 |
| Antioxydant | 0,05 |
| Isopropanol | 40,00 |
| Conservateur | 0,30 |
| Eau | qsp 100 % |

### Composition 5 : Crème (émulsion huile-dans-eau) pour les prurits anaux

| | |
|---|---|
| Spantide II | 5,00 |
| Cétyldiméthicone copolyol | 2,50 |
| NaCI | 0,60 |
| NaOH | qsp pH = 5 |
| Cyclométhicone | 18,00 |
| Polypropylèneglycol myristyl éther | |
| à 3 moles de propylène glycol | 6,00 |
| Glycérine | 3,00 |
| Conservateur | 0,20 |
| Eau | qsp 100 % |

### Composition 6 : Crème (émulsion huile-dans-eau) pour le traitement des prurits sévères

Cette composition se distingue de la composition 3 par la présence de 0,05 % de lidocaïne au lieu du 1 % d'acides lactique/acétique.

## Revendications

1. Utilisation d'au moins un antagoniste de substance P pour la préparation d'une composition pharmaceutique ou dermatologique, pour le traitement symptomatique des lichens, des prurigos, des toxidermies prurigineuses et des prurits sévères d'origine neurogène.

2. Utilisation selon la revendication 1, caractérisée en ce que l'antagoniste de substance P est choisi parmi les peptides et les composés comprenant au moins un hétérocycle et les composés azotés comprenant au moins un cycle benzénique.

3. Utilisation selon la revendication 1 ou 2, caractérisée en ce que le peptide est le sendide ou le spantide II.

4. Utilisation selon la revendication 1 ou 2, caractérisée en ce que le composé comprenant au moins un hétérocycle est un composé hétérocyclique azoté choisi parmi les dérivés de 2-tricyclyl-2-amino-éthane, les dérivés de spirolactame, les dérivés de quinuclidine, les dérivés azacycliques, les dérivés d'aminopyrrolidine, les dérivés de pipéridine, les aminoazahétérocycles, les dérivés d'isoindole.

5. Utilisation selon la revendication 1 ou 2, caractérisée en ce que le composé comprenant au moins un hétérocycle est un composé hétérocyclique oxygéné ou soufré choisi parmi les dérivés du furanne, les dérivés du benzofuranne, les dérivés du thiophène et les dérivés du benzothiophène, et notamment les tétrazolyl-benzofuranne-carboxamides ou les tétrazolyl-benzothiophène-carboxamides.

6. Utilisation selon la revendication 1 ou 2, caractérisée en ce que le composé azoté à au moins un cycle benzènique est un dérivé d'éthylène diamine.

7. Utilisation selon l'une quelconque des revendications précédentes, caractérisée en ce que l'antagoniste de substance P est utilisé en une quantité allant de 0,000001 à 5 % en poids par rapport au poids total de la composition.

8. Utilisation selon l'une quelconque des revendications précédentes, caractérisée en ce que l'antagoniste de substance P est utilisé en une quantité allant de 0,0001 à 0,1 % en poids par rapport au poids total de la composition.

9. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

10. Utilisation selon l'une quelconque des revendications précédentes, caractérisée en ce que la composition contient au moins un actif choisi parmi les protéines, les hydrolysats de protéine, les acides aminés, les polyols, l'urée, les sucres, les dérivés de sucre, les vitamines, l'amidon, les extraits végétaux, les hydroxyacides, les rétinoïdes, les acides gras essentiels, les céramides, les huiles essentielles, l'acide salicylique et ses dérivés.

## Patentansprüche

1. Verwendung mindestens eines Antagonisten der Substanz P für die Herstellung einer pharmazeutischen oder dermatologischen Zusammensetzung zur symptomatischen Behandlung von Lichen (Flechten), Prurigos, pruriginösen Toxikodermien und schwerem Pruritus neurogenen Ursprungs.

2. Verwendung nach Anspruch 1, dadurch gekennzeichnet, dass der Antagonist der Substanz P unter den Peptiden und den Verbindungen, die mindestens einen Heterocyclus enthalten und den stickstoffhaltigen Verbindungen, die mindestens einen Benzolring enthalten, ausgewählt ist.

3. Verwendung nach einem der Ansprüche 1 und 2, dadurch gekennzeichnet, dass es sich bei dem Peptid um Sendide oder Spantide II handelt.

4. Verwendung nach einem der Ansprüche 1 und 2, dadurch gekennzeichnet, dass die Verbindung, die mindestens einen Heterocyclus enthält, eine stickstoffhaltige heterocyclische Verbindung ist, die unter den 2-Tricyclyl-2-aminoethan-Derivaten, Spirolactam-Derivaten, Chinuclidin-Derivaten, azacyclische Derivaten, Aminopyrrolidin-Derivaten, Piperidin-Derivaten, Aminoazaheterocyclen, Isoindol-Derivaten ausgewählt ist.

5. Verwendung nach einem der Ansprüche 1 und 2, dadurch gekennzeichnet, dass die Verbindung, die mindestens einen Heterocyclus enthält, eine sauerstoffhaltige oder schwefelhaltige heterocyclische Verbindung ist, die unter Furan-Derivaten, Benzofuran-Derivaten, Thiophen-Derivaten und Benzothiophen-Derivate und insbesondere unter Tetrazolylbenzofurancarboxamiden und Tetrazolylbenzothiophencarboxamiden ausgewählt ist.

6. Verwendung nach Anspruch 1 oder 2, dadurch gekennzeichnet, dass die stickstoffhaltige Verbindung mit mindestens einem Benzolring ein Ethylendiamin-Derivat ist.

7. Verwendung nach einem der vorhergehenden Ansprüche, dadurch gekennzeichnet, dass der Antagonist der Substanz P in einer Menge von 0,000001 bis 5 Gew.-%, bezogen auf das Gesamtgewicht der Zusammensetzung, verwendet wird.

8. Verwendung nach einem der vorhergehenden Ansprüche, dadurch gekennzeichnet, dass der Antagonist der Substanz P in einer Menge von 0,0001 bis 0,1 Gew.-%, bezogen auf das Gesamtgewicht der Zusammensetzung, verwendet wird.

9. Verwendung nach einem der vorhergehenden Ansprüche, dadurch gekennzeichnet, dass die Zusammensetzung eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Vesikeldispersion, eine Dispersion von Mikrokapseln oder Mikropartikeln ist.

10. Verwendung nach einem der vorhergehenden Ansprüche, dadurch gekennzeichnet, dass die Zusammensetzung mindestens einen Wirkstoff enthält, der unter Proteine, Proteinhydrolysaten, Aminosäuren, Polyolen, Harnstoff, Zuckern, Zuckerderivaten, Vitaminen, Stärke, Pflanzenextrakten, Hydroxysäuren, Retinoiden, essentiellen Fettsäuren, Ceramiden, etherischen Ölen, Salicylsäure und ihren Derivaten ausgewählt ist.

## Claims

1. Use of at least one substance P antagonist for the preparation of a pharmaceutical or dermatological composition for the symptomatic treatment of lichens, prurigo, pruriginous toxicoderma and severe pruritus of neurogenic origin.

2. Use according to Claim 1, characterized in that the substance P antagonist is chosen from peptides and compounds comprising at least one heterocycle and nitrogen compounds comprising at least one benzene ring.

3. Use according to Claim 1 or 2, characterized in that the peptide is sendide or spantide II.

4. Use according to Claim 1 or 2, characterized in that the compound comprising at least one heterocycle is a heterocyclic nitrogen compound chosen from 2-tricyclyl-2-aminoethane derivatives, spirolactam derivatives, quinuclidine derivatives, azacyclic derivatives, aminopyrrolidine derivatives, piperidine derivatives, aminoazaheterocycles and isoindole derivatives.

5. Use according to Claim 1 or 2, characterized in that the compound comprising at least one heterocycle is a heterocyclic oxygen or sulphur compound chosen from furan derivatives, benzofuran derivatives, thiophene derivatives and benzothiophene derivatives, and in particular tetrazolylbenzofurancarboxamides or tetrazolylbenzothiophenecarboxamides.

6. Use according to Claim 1 or 2, characterized in that the nitrogen compound containing at least one benzene ring is an ethylenediamine derivative.

7. Use according to any one of the preceding claims, characterized in that the substance P antagonist is used in an amount ranging from 0.000001 to 5% by weight relative to the total weight of the composition.

8. Use according to any one of the preceding claims, characterized in that the substance P antagonist is used in an amount ranging from 0.0001 to 0.1% by weight relative to the total weight of the composition.

9. Use according to any one of the preceding claims, characterized in that the composition is an aqueous, oily or aqueous-alcoholic solution, a water-in-oil emulsion, an oil-in-water emulsion, a microemulsion, an aqueous gel, an anhydrous gel, a serum or a dispersion of vesicles, of microcapsules or of microparticles.

10. Use according to any one of the preceding claims, characterized in that the composition contains at least one active agent chosen from proteins, protein hydrolysates, amino acids, polyols, urea, sugars, sugar derivatives, vitamins, starch, plant extracts, hydroxy acids, retinoids, essential fatty acids, ceramides, essential oils and salicylic acid and its derivatives.
